# EUROPÄISCHE PATENTSCHRIFT

(11) **EP 2 091 641 B1**
(45) Veröffentlichungstag und Bekanntmachung des Hinweises auf die Patenterteilung: **23.06.2010**
(21) Anmeldenummer: 07816211.2
(22) Anmeldetag: 23.10.2007
(51) Int. Cl.: B01F 11/00, B01F 15/00, B01F 3/08, B01F 3/12, C12M 1/00, C12M 1/02, C12M 3/00

(54) **SCHWINGMISCHER**
VIBRATORY MIXER
MÉLANGEUR À AGITATION

(30) Priorität: 14.11.2006 CH 18272006
(43) Veröffentlichungstag der Anmeldung: 26.08.2009
(73) Patentinhaber: Röll, Marcel, 8124 Maur (CH)
(72) Erfinder: Röll, Marcel, 8124 Maur (CH)
(74) Vertreter: Schmauder & Partner AG Patent- & Markenanwälte VSP
(86) Internationale Anmeldenummer: PCT/CH2007/000528
(87) Internationale Veröffentlichungsnummer: WO 2008/058407

(56) Entgegenhaltungen:
- EP-A- 1 442 785
- WO-A-00/66706
- DE-A1- 1 444 466
- US-A1- 2002 197 713
- US-A1- 2006 013 063

## Beschreibung

### Technisches Gebiet

Die Erfindung betrifft einen Schwingmischer gemäss dem Oberbegriff von Anspruch 1. Der Schwingmischer ist besonders geeignet als so genannter Einwegmischer für biologische, chemische sowie Fermentations-Mischprozesse für ein Volumen von 10 bis 1000 Litern.

### Stand der Technik

Schwingmischer der vorstehend benannten Gattung sind seit langem bekannt, z.B. aus der WO-A-00/066706 oder auch - in einfacherer Weise - in der US-A-6190913 B1, der US-A-2002/197 713 oder in der US-A-2003/036192. In allen diesen Druckschriften wird für einen Bioreaktor bzw. Schwingmischer vorgeschlagen, einen Kunststoffbeutel auf einem Container oder auf einer schwingbaren Platte anzuordnen, mit dem Behandlungsgut zu befüllen und den Inhalt des Beutels durch einen Schwingvorgang der Platte zu durchmischen und damit reagieren zu lassen. In der EP-1 442 785 A1 wird bereits ein zur Schwingachse senkrechter Abwinkelbereich vorgeschlagen.

### Darstellung der Erfindung

Aufgabe der Erfindung ist es, einen Schwingmischer der genannten Art weiter zu verbessern.

Die gestellte Aufgabe wird gelöst durch einen Schwingmischer mit den Merkmalen des Anspruches 1. Dadurch, dass der Container einen Abwinkelbereich aufweist, welcher mit der Schwingachse zusammenfällt oder zu dieser im Wesentlichen parallel ist, wird die Auflagefläche in zwei Teilflächen unterteilt, die überdies einen Winkel von 60° bis 120° bilden. Dies führt zu einer verbesserte Durchmischung, die auch besser zu kontrollieren ist.

Etwaige Abweichungen des Abwinkelbereiches von der Schwingachse können 15° bis 20°, vorzugsweise unter 5° - betragen.

Vorteilhafte Ausgestaltungen der Erfindung sind in den Ansprüchen 2 bis 16 beschrieben.

Durch die Mittel zur Einstellung des Schwingantriebes kann der Schwingwinkel und die Schwinggeschwindigkeit den optimalen Prozessbedingungen (Mischen) angepasst werden. Es hat sich herausgestellt, dass sich das Schwingen von 50°-60° auf beide Seiten für den Mischprozess als vorteilhaft erweist. Der ganze Antrieb sollte somit um mindestens bis zu 120° schwingbar sein (Anspruch 2). Die Schwingachse kann vorteilhafterweise die Schwerpunktresultierenden des Containers schneiden, welches sich als Vorteil für die Antriebsleistung zeigt (Anspruch 3).

Der Container ist erfindungsgemäss um 60° bis 120° abgewinkelt. Es hat sich jedoch gezeigt, dass sich ein Winkel von 90° als ideal für den Mischprozess erweist (Anspruch 4). Das Fertigungsmaterial für den Container kann vorteilhafterweise Chromstahl sein; es kann jedoch auch ein Kunststoff verwendet werden. Der Container sollte ein Volumen von mindestens 50 Liter aufweisen. An den Schenkelenden des Containers ist eine Ausbuchtung angebracht, die als Flüssigkeitsreservoir dient und zugleich aber auch das Verschieben des Beutels im Container verhindert, wenn dieser über die horizontale Lage bewegt wird (Anspruch 5). Der Abwinkelbereich des Containers unterteilt den Beutel in zwei Abteilungen (Anspruch 6). Der Abwinkelbereich kann eine horizontale Fläche aufweisen, auf der Strömungsbrecher in beliebiger Form angebracht oder eingearbeitet sind (Anspruch 7 und 8). Diese Strömungsbrecher ergeben eine zusätzliche Verwirbelung der Flüssigkeit bei der Überströmung.

Durch das Anbringen einer in der Höhe einstellbaren Querstrebe direkt über dem Abwinkelbereich kann die Überströmgeschwindigkeit der Flüssigkeit bei den Strömungsbrechern eingestellt werden (Anspruch 9).

Zur Befestigung des Beutels können vorteilhafterweise an zumindest einem, zweckmässigerweise an beiden Enden des Abwinkelbereiches Befestigungsbolzen angebracht sein, in welchen der Beutel eingehängt ist (Anspruch 10).

Der Beutel besteht vorteilhafterweise aus einer Kunststofffolie vorzugsweise aus PE, EVA, Polyester. Die Form des Beutels wird dabei der Innenform oder dem Volumen des Containers angepasst. Die Beutellänge reicht über die gesamte abwickelte Länge des Containers.

Vorteilhaft ist es, wenn der Beutel mit einer Einweg pH Sonde und - bei Bedarf - noch weiteren Einweg-Sonden zur Überwachung des Prozesses ausgerüstet ist (Anspruch 11).

In der Mittellinie des Beutels sind an den beiden Seiten Laschen mit Positionslöchern angebracht, die zur genauen Positionierung und Befestigung des Beutels im Container dienen (Anspruch 12). Vorteilhaft ist es, durch das Anbringen eines Filtergewebes im Innenbereich des Beutels, welches bei den beiden Schenkelseiten positioniert ist, und durch Zuführen eines Gases zwischen das Filtergewebe und der vertikal überströmten Filterfläche einen zusätzlichen Gaseintrag in die Flüssigkeit zu gewährleisten (Wasserfalleffekt gemäss Anspruch 13).

Besonders vorteilhaft ist der Schwingmischer, wenn er zusätzlich eine Einrichtung zum Bestimmen des Inhaltes des Beutels aufweist, um damit den Schwingwinkel und/oder die Schwinggeschwindigkeit zu steuern. Wenn nämlich der Beutel einen grossen Inhalt aufweist, dann ist nur ein relativ kleiner Schwingwinkel notwendig, um einen Mischvorgang durchzuführen zu können. Wenn aber z.B. bereits ein grosser Teil des Inhalts entnommen wurde, dann verbleibt der Rest im unteren Bereich und es wird ein grosser Schwingwinkel notwendig sein, um diesen Rest über den Biegebereich zu bringen. Diese Einrichtung wird vorzugsweise ein Managementsystem sein, mit dem die Beladung des Beutels und die Entnahme verarbeitet werden (Anspruch 14). Vorteilhafterweise wird aber zumindest auch ein Gewichtssensor zum Messen des Gewichts des Containers (brutto) und damit des Beutelinhalts (netto) zum Einsatz kommen, der vorteilhafterweise in der Nähe bzw. unter dem Biegebereich angeordnet ist (Anspruch 15).

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können.

### Kurze Beschreibung der Zeichnungen

Nachfolgend werden Ausführungsbeispiele der Erfindung an Hand der Zeichnungen näher beschrieben, dabei zeigen:
- Figur 1: einen Schwingmischer in neutraler Position, dargestellt von der Sei- te;
- Figur 2: einen Container für den Schwingmischer von Figur 1;
- Figur 3: den Schwingmischer von Figur 1, in nach links geschwenkter Stel- lung, wobei sich der rechte Containerteil in horizontaler Stellung be- findet;
- Figur 4: den Schwingmischer von Figur 1, in nach rechts geschwenkter Stel- lung, wobei sich der linke Containerteil in horizontaler Stellung be- findet;
- Figur 5: einen Beutel (Mischbeutel) für den Container aus Figur 1, in Ansicht von oben;
- Figur 6: den Beutel (Mischbeutel) für den Container aus Figur 1, in Ansicht von der Seite;
- Figur 7: das Funktionsprinzip des Strömungsbrechers für den Schwingmi- scher der Figur 1; und
- Figur 8: den Schwingmischer der Figur 1, in perspektivischer Darstellung.

### Wege zur Ausführung der Erfindung

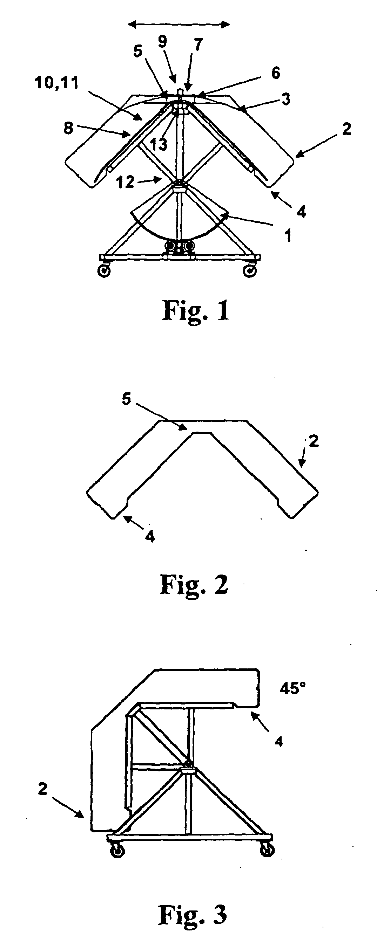

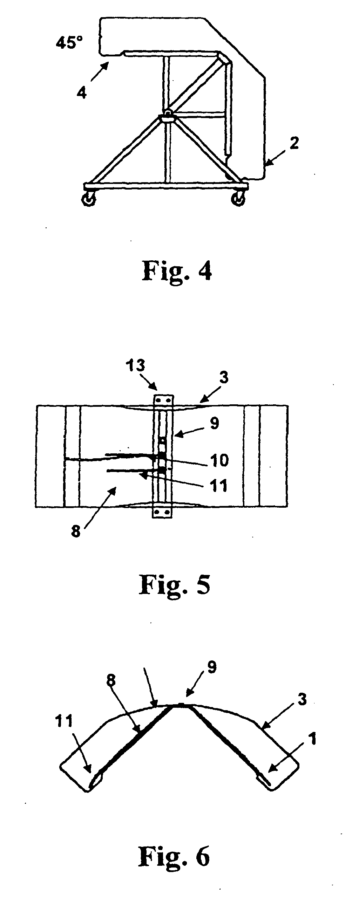

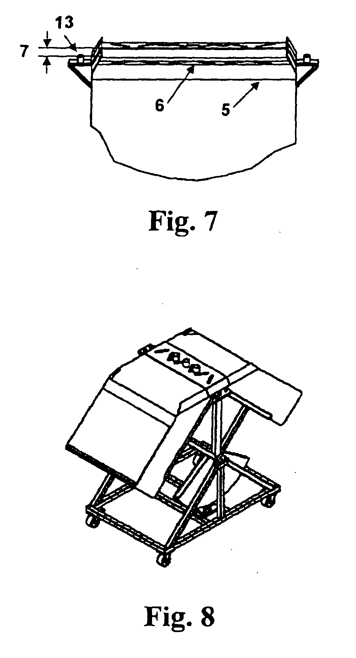

Der Schwingmischer gemäss den Figuren 1 bis 8 ist ein 50 bis 1000 Liter Medien- und Kultivierungs- Mischsystem und beruht auf induzierter Flüssigkeitsumlagerung. Das System verfügt über einen Schwingmechanismus 1, der eine bis zu 120 Grad reichende Schwingbewegung ausführen kann. Auf diesem Schwingmechanismus ist ein abgewinkelter Container 2 angebracht, in den ein der Containerform angepasster Beutel 3 - im vorliegenden Ausführungsbeispiel ein Kunststoffbeutel - eingelegt ist. Der Container ist vorzugsweise von 60° bis 120°, im in den Figuren dargesellten Ausführungsbeispiel um 90° abgewinkelt; dies ergibt die wichtige Formgebung für den Mischprozess. Der Beutel 3 besteht vorzugsweise aus einer Kunststofffolie aus Polyäthylen, EVA, Polyester oder einem ähnlichen Material und wird in den Container 2 eingelegt. Der Beutel 3 dient zusätzlich als Einweghülle, die im vorliegenden Ausführungsbeispiel vorsterilisiert ist. Der gesamte Misch- und Reaktionsprozess findet in diesem Beutel 3 statt und eliminiert die Notwendigkeit der Reinigung des Containers 2 nach dem Ende des Prozesses.

Der Beutel ist mit einem Schraubverschluss 9 sowie einem Anschlussschlauch 10, ferner mit einem Entnahmeschlauch 11 mit einem innen liegenden Ansaugschlauch und einem Probeentnahmeanschluss ausgerüstet. Eine Einwegsonde zur Messung des pH-Wertes und weitere Einweg-Sonden dienen zur Überwachung des Prozesses.

Der Container 2 ist an den Enden mit Ausbuchtungen 4 versehen, so dass sich der Beutel 3 mit der sich in diesen Ausbuchtungen sammelnden Flüssigkeit fixiert und zugleich ein Flüssigkeitsreservoir bildet, aus welchem während des Schwingens kontinuierlich Flüssigkeit entnommen werden kann.

Durch das Schwingen des mit Flüssigkeit gefüllten Beutels im Containers 2 aus der neutralen Lage bis zu 60° auf beide Seiten, so dass der eine oder andere Containerteil abwechslungsweise in die horizontale Lage - im hier beschriebenen Ausführungsbeispiel bei einem Schwingen von jeweils 45° - gebracht wird, entsteht im Abwinkelbereich 5 eine Flüssigkeitsüberströmung. Mit der einstellbaren Schwinggeschwindigkeit kann die Flüssigkeit somit kontrolliert gemischt werden.

Das Einstellen der Schwinggeschwindigkeit und das Anbringen von Strömungsbrechern 6 im Abwinkelbereich 5 sowie das Verengen des Beutelquerschnittes 7 über dem Abwinkelbereich verursacht ein mehr oder weniger starkes Verwirbeln (Mischen). Das Beschleunigen der Flüssigkeit über den Abwinkelbereich 5 und das erneute Abbremsen am Boden des Containers 2 ergibt weitere für den Prozess wichtige Mischturbulenzen.

Das Umlagern der gesamten Flüssigkeit pro Schwingeinheit erzeugt einen maximalen Mischwert in Verbindung mit einem Einweg-Beutel, somit wird auch eine maximale Sterilität und Reinigungsperformance garantiert.

Das Anbringen eines Filtergewebes 8 im Innenbereich des Beutels, welches an beiden Schenkelseiten positioniert ist, ermöglicht das Zuführen eines Gases zwischen das Filtergewebe und der vertikal überströmten Filterfläche. Folglich kann ein zusätzlicher Gaseintrag in die Flüssigkeit gewährleistet werden (Wasserfalleffekt).

Zusätzlich weist der Schwingmischer eine Einrichtung zum Bestimmen des Inhaltes des Beutels auf, um damit den Schwingwinkel und/oder die Schwinggeschwindigkeit zu steuern. Wenn der Beutel einen grossen Inhalt aufweist, wird ein relativ kleiner Schwingwinkel vorgegeben, um einen Mischvorgang durchzuführen. Wenn aber der Beutel nur einen kleinen Inhalt aufweist, wird ein grosser Schwingwinkel vorgegeben, um diesen Rest des Mischgutes über den Abwinkelbereich zu bringen. Im vorliegenden Ausführungsbeispiel weist der Schwingmischer einen Gewichtssensor auf, der in der Nähe bzw. unter dem Abwinkelbereich angeordnet ist.

Im vorliegenden Ausführungsbeispiel sind die Teilflächen des Containers einzeln beheizbar, um die Reaktionsparameter steuern bzw. regeln zu können.

### Bezugszeichenliste

- 1: Schwingmechanismus
- 2: Container
- 3: Beutel
- 4: Ausbuchtung
- 5: Abwinkelbereich
- 6: Strömungsbrecher
- 7: Beutelquerschnitt
- 8: Filtergewebe
- 9: Schraubanschluss
- 10: Anschlussschlauch
- 11: Entnahmeschlauch
- 12: Schwerpunktresultierende
- 13: Befestigungsbolzen

## Patentansprüche

1. Schwingmischer für biologische, chemische sowie Fermentations-Mischprozesse, mit einem Reaktorgestell und mit einem Container, wobei der Container (2) eine Auflagefläche aufweist, auf der ein Beutel (3) aufliegt, wobei der Container (2) mittels eines Schwingmechanismus (1) um eine im Wesentlichen horizontale Schwingachse des Reaktorgestells schwingbar ist und wobei der Schwingmischer Mittel zur Einstellung der Grösse des Schwingwinkels aufweist, **dadurch gekennzeichnet, dass** der Container (2) zumindest einen Abwinkelbereich (5) aufweist, dessen Längsachse im Wesentlichen zur Schwingachse ausgerichtet ist und der die Auflagefläche in zwei Teilflächen unterteilt, die einen Winkel von 60° bis 120° zueinander aufweisen.

2. Schwingmischer nach Anspruch 1, **dadurch gekennzeichnet, dass** der Container (2) durch den Schwingmechanismus (1) um einen Winkel von mindestens 90°, vorzugsweise von 120° schwingbar ist.

3. Schwingmischer nach Anspruch 1 oder 2, **dadurch gekennzeichnet, dass** die Schwingachse die Schwerpunktresultierenden (12) des Containers (2) schneidet.

4. Schwingmischer nach einem der vorstehenden Ansprüche, **dadurch gekennzeichnet, dass** die Teilflächen durch den Abwinkelbereich (5) einen Winkel von ca. 90° aufweist.

5. Schwingmischer nach einem der vorstehenden Ansprüche, **dadurch gekennzeichnet, dass** an den Schenkelenden des Containers (2) eine Ausbuchtung (4) angebracht ist, die ein Flüssigkeitsreservoir ausbildet.

6. Schwingmischer nach einem der vorstehenden Ansprüche, **dadurch gekennzeichnet, dass** der Abwinkelbereich (5) des Containers (2) so ausgebildet ist, dass er den Beutel (3) in zwei Abteilungen unterteilt.

7. Schwingmischer nach einem der vorstehenden Ansprüche, **dadurch gekennzeichnet, dass** der Abwinkelbereich (5) eine horizontale Fläche aufweist.

8. Schwingmischer nach einem der vorstehenden Ansprüche, **dadurch gekennzeichnet, dass** im Bereich des Abwinkelbereichs (5) Strömungsbrecher (6) angebracht oder eingearbeitet sind.

9. Schwingmischer nach Anspruch 8, **dadurch gekennzeichnet, dass** eine in der Höhe einstellbare Querstrebe im Bereich des Abwinkelbereichs (5) angeordnet ist.

10. Schwingmischer nach einem der vorstehenden Ansprüche, **dadurch gekennzeichnet, dass** an zumindest einem Ende des Abwinkelbereichs (5) Befestigungsbolzen (13) angeordnet sind, in die der Beutel (3) einhängbar ist.

11. Schwingmischer nach einem der vorstehenden Ansprüche, **dadurch gekennzeichnet, dass** der Beutel (3) zumindest eine Einweg pH Sonde zur Überwachung des Mischprozesses aufweist.

12. Schwingmischer nach einem der vorstehenden Ansprüche, **gekennzeichnet durch** Laschen mit Positionslöchern, die in der Mittellinie des Beutels (3) an den beiden Seiten angebracht sind, zur Positionierung und Befestigung des Beutels (3) im Container (2).

13. Schwingmischer nach einem der vorstehenden Ansprüche, **gekennzeichnet durch** ein Filtergewebe (8) im Innenbereich des Beutels (3), welches vorzugsweise an den beiden Schenkelseiten positioniert ist, und eine Einrichtung zum Zuführen eines Gases zwischen das Filtergewebe (8) und der vertikal überströmten Filterfläche.

14. Schwingmischer nach einem der vorstehenden Ansprüche, **gekennzeichnet durch** eine Einrichtung zum Bestimmen des Inhaltes des Beutels, mittels der der Schwingwinkel und/oder die Schwinggeschwindigkeit steuerbar ist, wobei die Einrichtung vorzugsweise ein Managementsystem umfasst, mittels dessen die Beladung des Beutels (3) und die Entnahme des Beutels (3) verarbeitbar ist.

15. Schwingmischer nach Anspruch 14, **dadurch gekennzeichnet, dass** die Einrichtung einen Gewichtssensor zur Gewichtsmessung des Containers (2) und/oder des Beutels (3) umfasst.

## Claims

1. A vibratory mixer for biological, chemical and fermentation mixing processes, with a reactor stand and with a container, the container (2) having a bearing surface on which a bag (3) lies, the container (2) being vibratable about an essentially horizontal vibratory axis of the reactor stand by means of a vibratory mechanism (1), and the vibratory mixer having means for setting the magnitude of the vibratory angle, **characterized in that** the container (2) has at least one angled region (5), the longitudinal axis of which is essentially aligned to the vibratory axis and which subdivides the bearing surface into two subsurfaces which are at an angle of 60° to 120° with respect to one another.

2. The vibratory mixer as claimed in claim 1, **characterized in that** the container (2) is vibratable by the vibratory mechanism (1) at an angle of at least 90°, preferably of 120°.

3. The vibratory mixer as claimed in claim 1 or 2, **characterized in that** the vibratory axis intersects the center of gravity resultant (12) of the container (2).

4. The vibratory mixer as claimed in one of the preceding claims, **characterized in that** the subsurfaces due to the angled region (5) have an angle of approximately 90°.

5. The vibratory mixer as claimed in one of the preceding claims, **characterized in that** a protuberance (4) which forms a liquid reservoir is provided at the leg ends of the container (2).

6. The vibratory mixer as claimed in one of the preceding claims, **characterized in that** the angled region (5) of the container (2) is designed such that it subdivides the bag (3) into two compartments.

7. The vibratory mixer as claimed in one of the preceding claims, **characterized in that** the angled region (5) has a horizontal surface.

8. The vibratory mixer as claimed in one of the preceding claims, **characterized in that** flow breakers (6) are attached or incorporated in the region of the angled region (5).

9. The vibratory mixer as claimed in claim 8, **characterized in that** a vertically adjustable transverse strut is arranged in the region of the angled region (5).

10. The vibratory mixer as claimed in one of the preceding claims, **characterized in that** fastening bolts (13) in which the bag (3) can be suspended are arranged at at least one end of the angled region (5).

11. The vibratory mixer as claimed in one of the preceding claims, **characterized in that** the bag (3) has at least one disposable pH probe for monitoring the mixing process.

12. The vibratory mixer as claimed in one of the preceding claims, **characterized by** tabs with position holes, which are formed on both sides on the center line of the bag (3), for positioning and fastening the bag (3) in the container (2).

13. The vibratory mixer as claimed in one of the preceding claims, **characterized by** a filter fabric (8) in the internal region of the bag (3), which filter fabric is preferably positioned on the two leg sides, and by a device for feeding a gas between the filter fabric (8) and the vertical-overflow filter surface.

14. The vibratory mixer as claimed in one of the preceding claims, **characterized by** a device for determining the capacitys of the bag, by means of which device the vibratory angle and/or the vibratory speed can be controlled, wherein the device comprises a management system, by means of which the loading of the bag (3) and the extraction of the bag (3) can be processed.

15. The vibratory mixer as claimed in claim 14, **characterized in that** the device comprises a weight sensor for measuring the weight of the container (2) and/or of the bag (3).

## Revendications

1. Mélangeur-agitateur pour des procédés de mélanges biologiques, chimiques et de fermentation, comportant un châssis de réacteur et un conteneur,
- le conteneur (2) ayant une surface d'appui recevant une poche (3),
- le conteneur (2) étant mis en oscillation principalement autour d'un axe d'oscillation horizontal du châssis du réacteur à l'aide d'un mécanisme d'oscillation (1), et
- le mélangeur-agitateur comporte des moyens pour régler l'amplitude de l'angle d'oscillation,
**caractérisé en ce que**
le conteneur (2) comporte au moins une plage pliée descendante (5) dont l'axe longitudinal est aligné essentiellement sur l'axe d'oscillation et y subdivise la surface d'appui en deux surfaces partielles faisant entre elles un angle de 60° à 120°.

2. Mélangeur-agitateur selon la revendication 1,
**caractérisé en ce que**
le conteneur (2) est mis en oscillation par un mécanisme d'oscillation (1) suivant un angle d'au moins 90° et de préférence de 120°.

3. Mélangeur-agitateur selon la revendication 1 ou 2,
**caractérisé en ce que**
l'axe d'oscillation coupe les résultantes du centre de gravité (12) du conteneur (2).

4. Mélangeur-agitateur selon l'une des revendications précédentes,
**caractérisé en ce que**
les surfaces partielles dans la plage pliée descendante (5), font un angle d'environ 90°.

5. Mélangeur-agitateur selon l'une des revendications précédentes,
**caractérisé en ce que**
les extrémités des branches du conteneur (2) comportent une excroissance (4) formant un réservoir à liquide.

6. Mélangeur-agitateur selon l'une des revendications précédentes,
**caractérisé en ce que**
la plage pliée descendante (5) du conteneur (2), est réalisée pour subdiviser la poche (3) en deux compartiments.

7. Mélangeur-agitateur selon l'une des revendications précédentes,
**caractérisé en ce que**
la plage pliée descendante (5), comporte une surface horizontale.

8. Mélangeur-agitateur selon l'une des revendications précédentes,
**caractérisé en ce qu'**
au niveau de la plage pliée descendante (5), on a un brise-jet (6) installé ou usiné.

9. Mélangeur-agitateur selon la revendication 8,
**caractérisé par**
une entretoise de hauteur réglable prévue au niveau de la plage pliée descendante (5).

10. Mélangeur-agitateur selon l'une des revendications précédentes,
**caractérisé en ce qu'**
à au moins une extrémité de la plage pliée descendante (5), il y a des goujons de fixation (13) auxquels s'accroche la poche (3).

11. Mélangeur-agitateur selon l'une des revendications précédentes,
**caractérisé en ce que**
la poche (3) comporte au moins une sonde à pH, à usage unique, pour surveiller le procédé de mélange.

12. Mélangeur-agitateur selon l'une des revendications précédentes,
**caractérisé par**
des pattes avec des orifices de positionnement prévus dans la ligne d'axe de la poche (3), sur les deux côtés, pour positionner et fixer la poche (3) dans le conteneur (2).

13. Mélangeur-agitateur selon l'une des revendications précédentes,
**caractérisé par**
un tissu filtrant (8) prévu dans la zone intérieure de la poche (3), et positionné de préférence sur les deux côtés des branches et une installation pour introduire un gaz entre le tissu de filtre (8) et la surface de filtre verticale, sur laquelle déborde verticalement le flux.

14. Mélangeur-agitateur selon l'une des revendications précédentes,
**caractérisé par**
une installation pour déterminer le contenu de la poche, à l'aide de laquelle se commande l'angle d'oscillation et/ou la vitesse d'oscillation, l'installation comportant de préférence un système de gestion permettant de traiter la charge de la poche (3) et l'enlèvement de la poche (3).

15. Mélangeur-agitateur selon la revendication 14,
**caractérisé en ce que**
l'installation comporte un capteur de pesée pour peser le conteneur (2) et/ou la poche (3).
